# EUROPÄISCHE PATENTSCHRIFT

(11) **EP 0 612 316 B1**
(45) Veröffentlichungstag und Bekanntmachung des Hinweises auf die Patenterteilung: **05.02.1997**
(21) Anmeldenummer: 92923042.3
(22) Anmeldetag: 03.11.1992
(51) Int. Cl.: C07D 231/12

(54) **VERFAHREN ZUR HERSTELLUNG VON 3(5)-METHYLPYRAZOLEN**
PROCESS FOR PREPARING 3(5)-METHYLPYRAZOLES
PROCEDE DE FABRICATION DE 3(5)-METHYLPIRAZOLS

(30) Priorität: 11.11.1991 DE 4137011
(43) Veröffentlichungstag der Anmeldung: 31.08.1994
(73) Patentinhaber: BASF Aktiengesellschaft, 67063 Ludwigshafen (DE)
(72) Erfinder: KAESTNER, Ralf, D-6700 Ludwighafen (DE); RITTINGER, Stefan, D-6700 Ludwigshafen (DE); PAESSLER, Peter, D-6700 Ludwigshafen (DE); RIEBER, Norbert, D-6800 Mannheim 51 (DE)
(86) Internationale Anmeldenummer: EP9202514
(87) Internationale Veröffentlichungsnummer: WO9310098

(56) Entgegenhaltungen:
- EP-A- 0 274 600
- DE-A- 2 157 537
- DE-B- 1 222 910
- FR-A- 748 193
- US-A- 2 900 044
- US-A- 2 941 020
- US-A- 3 325 557
- JOURNAL OF ORGANIC CHEMISTRY. Bd. 34, Nr. 4, 1969, EASTON US Seiten 999 - 1001 W. PAUDLER; A.G. ZEILER '1,3-Butadiynes in the synthesis of heterocyclic compounds' in der Anmeldung erw hnt
- CHEMICAL ABSTRACTS, vol. 79, 1973, Columbus, Ohio, US; abstract no. 126390b, S.G. MATSOYAN ET AL. '3(5)-methylpyrazole' Seite 400 ;
- CHEMICAL ABSTRACTS, vol. 79, 1973, Columbus, Ohio, US; abstract no. 105131c, R.Y. MUSHII ET AL. 'Use of diacetylene from acetylene production waste gases for preparation of commercial products' Seite 418 ;
- CHEMICAL ABSTRACTS, vol. 94, 1981, Columbus, Ohio, US; abstract no. 174287e, V.N. STEPANOVA 'Isolation of acetylene and diacetylene from hydrocarbon gases' Seite 657 ;
- CHEMICAL ABSTRACTS, vol. 93, 1980, Columbus, Ohio, US; abstract no. 45861t, Z.A. MAMADZHANYAN ET AL. 'Improvement of the additional removal of diacetylene from pyrolytic acetylene' Seite 855 ;

## Beschreibung

Die vorliegende Erfindung betrifft ein Verfahren zur Herstellung von 3(5)-Methylpyrazolen aus Diacetylen und Hydrazinen.

3-Methylpyrazol und seine Derivate sind als Nitrifikationsinhibitoren für ammoniumhaltige Düngemittel von Bedeutung (vgl. z.B. US 3 635 690 und DE 27 45 833 A1).

N-substituierte Pyrazole sind von industrieller Bedeutung als Bestandteile von zahlreichen biologisch aktiven Wirksubstanzen (s. z.B.: EP 286969-A; EP 320 750 A; EP 234045-B1, EP 0269806-B1).

Verfahren zur Herstellung von 3(5)-Methylpyrazol aus Diacetylen und Hydrazin im Labormaßstab sind bekannt (Schroth et al., Z. Chem. 9, (1969), 110; Paudler et al., J. Org. Chem., 34, (1969), 999).

Im Stand der Technik wird darauf hingewiesen, daß die Handhabung von Diacetylen im technischen Maßstab äußerst problematisch ist (s. z.B. DE-AS 1 222 910) und Diacetylen daher nahezu ausschließlich zur Herstellung von Laborpräparaten in entsprechend kleinen Mengen genutzt wird (EP 274 600 A1).

In Zh. Prik. Khim. 44 (1971), 1921 wird ebenfalls die Reaktion von Diacetylen mit Hydrazinhydrat beschrieben. Bei der in dieser Literaturstelle empfohlenen Reinigung von Pyrolysegasen (Spaltgas) mit Hilfe dieser Reaktion treten jedoch folgende Probleme auf: Das Spaltgas enthält im allgemeinen nur ≤ 0,5 % ungesättigte Kohlenwasserstoffe mit drei oder mehr Kohlenstoffatomen. Damit ist das Spaltgas als Diacetylenquelle für Synthesen wenig geeignet, da die Konzentration des Diacetylens so gering ist, daß sehr große Gasmengen umzusetzen sind. Zudem hat das Spaltgas noch keinen Reinigungsprozess durchlaufen, so daß aufgrund des ungünstigen Verteilungsprofils an höheren Acetylenen zahlreiche Nebenprodukte resultieren. Darüber hinaus wird das Spaltgas mit Hydrazin verunreinigt, so daß in der Acetylenreinigung ein zusätzlicher Prozeßschritt notwendig wird (Waschturm zur Absorption von gestripptem Hydrazin).

Zur Überwindung derartiger Probleme ist aus der DE-OS 21 57 537 ein Verfahren zur gefahrlosen Handhabung niedrigmolekularer Acetylene bekannt, bei dem diese Acetylene an Aktivkohle adsorbiert und in dieser Form für die weitere Verwendung eingesetzt werden.

Dieses Verfahren ist äußerst umständlich und ein kontinuierlicher Prozeß aufgrund des Adsorptionsmittels verfahrenstechnisch problematisch.

Die EP 274 600 A1 offenbart ein Verfahren zur Umsetzung von Diacetylen mit Alkoholen, wobei die Durchführung im technischen Maßstab durch den Zusatz bestimmter höherer Kohlenwasserstoffe ermöglicht werden soll.

Der vorliegenden Erfindung liegt die Aufgabe zugrunde, ein Verfahren zur Herstellung von 3(5)-Methylpyrazolen der allgemeinen Formeln Ia und Ib 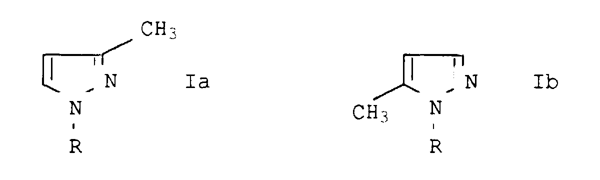 in der R für Wasserstoff, einen C₁-C₆-Alkylrest oder einen gegebenenfalls substituierten Phenylrest steht,

zur Verfügung zu stellen, bei dem Diacetylen ohne verfahrenstechnische Probleme in einfacher Weise mit Hydrazinen umgesetzt werden kann.

Es wurde nun gefunden, daß diese Aufgabe dadurch gelöst werden kann, daß
a) aus dem bei der Acetylenerzeugung resultierenden Spaltgas ein Teilstrom, der das Diacetylen enthält, durch reversible Absorption in einer polaren Flüssigkeit oder einem Gemisch höhersiedender Kohlenwasserstoffe abgetrennt und gegebenenfalls mit einem Verdünnungsmittel verdünnt wird und
b) dieser Teilstrom ohne weitere physikalische oder chemische Behandlung mit Hydrazinen der allgemeinen Formel II

   RHN-NH₂ II

   in der R die oben angegebene Bedeutung hat, bei Temperaturen von (-40) bis +150°C und Normaldruck umgesetzt wird.

Überraschenderweise läßt sich der beim technisch üblicherweise praktizierten Verfahren zur Spaltgasaufarbeitung anfallende, die höheren Acetylene enthaltende Teilstrom ohne weitere physikalische oder chemische Behandlung unmittelbar mit Hydrazinen in sehr guter Ausbeute zu 3(5)-Methylpyrazolen umsetzen. Die Ausbeuten liegen dabei in unerwarteter Weise wesentlich höher als in der Literatur für Umsetzungen mit reinem Diacetylen beschrieben (vgl. Veröffentlichungen von Paudler und Schroth, aaO, 73 und 80 %).

Die Umsetzung läßt sich formelmäßig wie folgt darstellen: 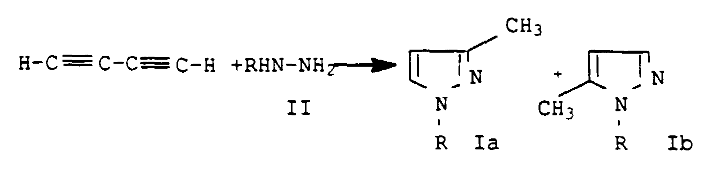 wobei R gleich Wasserstoff, C₁-C₆-Alkyl oder ein gegebenenfalls substituierter Phenylrest ist. Die Verbindungen der Formeln Ia und Ib werden hier zusammenfassend als 3(5)-Methylpyrazole bezeichnet.

Die technisch ausgeübten Varianten zur Erzeugung und Aufbereitung des erfindungsgemäß verwendeten Spaltgases ("crackgas") ist wohlbekannt (s. Ullmanns Encyclop. of Industr. Chem., V. Ed., Al, 1985, 97 ff, 111, Figure 13), so daß diese Prozesse hier nur kurz charakterisiert werden:
Kohlenwasserstoffe (z.B. Erdgas oder auch höhersiedende Fraktionen) werden bei den für die Acetylenentstehung notwendigen, hohen Temperaturen gespalten und die resultierenden Produkte werden unmittelbar nach der Reaktionszone (Spaltzone) durch Eindüsen einer Flüssigkeit (z.B. Wasser und Öl) rasch abgekühlt ("Quench"). Die Zusammensetzung des Spaltgases ist abhängig von den zur Spaltung eingesetzten Ausgangsstoffen, sowie den Spaltungsbedingungen.

Die erfindungsgemäß verwendeten Teilströme fallen bei der Zerlegung des Spaltgases durch eine Serie von physikalischen Trennvorgängen (bevorzugt durch Absorptions-/Desorptionsprozesse in einer Serie von Wasch- und Stripkreisläufen) in mehrere Teilströme an. Kennzeichnend für die verwendbaren, höheren Acetylene enthaltenden Teilströme ist, daß sie Diacetylen gegenüber dem Spaltgas in deutlich höheren Konzentrationen (angereichert) enthalten.

Es ist bevorzugt, daß die erfindungsgemäß eingesetzten Teilströme ein Verdünnungsmittel enthalten oder daß den Teilströmen ein Verdünnungsmittel zugesetzt wird. Das Verdünnungsmittel kann flüssig oder gasförmig sein.

Als Verdünnungsmittel geeignet sind allgemein polare Flüssigkeiten, etwa organische Lösungsmittel wie Alkohole, insbesondere Methanol, Ketone, N-Methylpyrrolidon und/oder Dimethylformamid. Auch Wasser ist ggfs. im Gemisch mit den genannten polaren Flüssigkeiten, einsetzbar. In Frage kommen auch Gemische höhersiedender Kohlenwasserstoffe, Benzol, Toluol oder Xylole.

Das flüssige Verdünnungsmittel kann das im Verfahrensschritt a) verwendete Absorptionsmittel sein.

Der Rest R steht bevorzugt für Wasserstoff, d.h. bevorzugt wird unsubstituiertes Hydrazin eingesetzt. Die dabei entstehenden Methylpyrazole der allgemeinen Formeln Ia und Ib mit R = H stehen in einem tautomeren Gleichgewicht, d.h. sie wandeln sich bei Raumtemperatur ineinander um.

R kann aber auch für einen (C₁-C₆)-Alkylrest und für einen gegebenenfalls substituierten Phenylrest stehen. Als Substituenten am Phenylrest sind eine Vielzahl von Resten möglich, wie z.B. Hydroxy, Halogen (insbesondere F), Cyano, Nitro, (C₁-C₄)-Alkyl, (C₁-C₄)-Halogenalkyl, (C₁-C₆)-Alkoxy- (C₁-C₄)-Alkyl, (C₁-C₆)-Alkoxy, (C₁-C₆)-Alkoxy-(C₁-C₄)-Alkoxy, (C₁-C₆)-Halogenalkoxy, (C₁-C₄)-Alkylthio, (C₁-C₄)-Halogenalkylthio, Carboxy, (C₁-C₄)-Alkoxycarbonyl, (C₁-C₄)-Alkylsulfinyl, (C₁-C₄)-Halogenalkylsulfinyl, (C₁-C₄)-Alkylsulfonyl, (C₁-C₄)-Halogenalkylsulfonyl, (C₁-C₄)-Alkylsulfonyloxy, Halogen(C1-C4)-alkylsulfonyloxy, Phenyl, Halogenphenyl, Phenoxy, Halogenphenoxy.

Durch das erfindungsgemäße Verfahren wird eine explosionssichere Handhabung und Umsetzung von Diacetylen mit Hydrazinen im technischen Maßstab gewährleistet. Dies ist besonders vorteilhaft in den Verfahren, in denen die aus der Spaltgasaufbereitung stammenden, höheren Acetylene enthaltenden Teilströme ansonsten verbrannt werden müssen, oder lediglich zur Entsorgung in den Crackprozeß zurückgeführt werden (s. Ullmann, aaO).

Sowohl flüssige Einsatzströme als auch gasförmige, durch Strippen (Desorption) der Waschlösungen erhältliche Teilströme zeichnen sich gegenüber dem Spaltgas durch ein für die gewünschte Umsetzung zu Methylpyrazolen günstigeres Verteilungsprofil der höheren Acetylene aus. Es sind nur noch unwesentliche Mengen anderer, höherer Acetylene (Pentadiin, Hexadiin, etc.) enthalten, da diese vorher abgeschieden werden. Dies führt zu einer hohen Reinheit der erhaltenen Methylpyrazole.

Die Abtrennung des das Diacetylen enthaltenden Teilstroms aus dem Spaltgas erfolgt in bevorzugter Weise so, daß die höheren Acetylene in einer Vorwäsche des das Gesamtacetylen enthaltenden Spaltgases mit einer Flüssigkeit absorbiert werden. Diese mit Diacetylen angereicherte Waschlösung kann dann noch in einem Vakuumstripper entgast werden, wobei die Gasfraktion nochmals vor oder unmittelbar nach einer eventuellen Kompression durch Zumischen eines Inertgasanteils verdünnt, d.h. stabilisiert wird.

Aus diesem Gasstrom durch Kondensation gewinnbare diacetylenhaltige Kohlenwasserstoffmischungen (sog. BTX-Fraktion) eignen sich gleichermaßen vorteilhaft für die beschriebene Umsetzung von Diacetylen zu Methylpyrazolen. Diese Vorgehensweise erbringt einen zusätzlichen Vorteil, da diese BTX-Kondensate aufgrund ihres Diacetylengehaltes üblicherweise nicht einer technischen Verwertung, abgesehen von der Verbrennung, zugeführt werden können. Nach Umsetzung des Diacetylens zu Methylpyrazolen ist als erste Kopffraktion diacetylenfreies BTX erhältlich, welches als Rohstoff, z.B. zur Herstellung von Benzol, verwendet werden kann.

Die bevorzugten Absorptionsmittel in der Spaltgasaufbereitung sind hochsiedende Kohlenwasserstoffe oder polare Flüssigkeiten, wie N-Alkyllactame mit C₁-C₃-Alkyl, insbesondere N-Methylpyrrolidon, C₁-C₅-Alkohole, insbesondere CH₃OH, Säureamide, insbesondere Dimethylformamid, alkylierte zyklische Harnstoffe, insbesondere Dimethylpropylenharnstoff, Wasser, C₁-C₆-Amine oder NH₃.

Ein typischer flüssiger, höhere Acetylene enthaltender Teilstrom hat folgende Zusammensetzung: 32 % Benzol, 28 % Toluol, 17 % Xylol (BTX), 8 % Styrol, 6 % Diacetylen. In einem weiteren Beispiel setzt sich ein Strom aus MeOH (89 %), Acetylen (0,6 %), Diacetylen (2,2 %), Vinylacetylen (0,4 %), Cyclopentadien (1 %), Benzol (1,8 %) und Toluol (1,3 %) zusammen.

Zur Verdünnung des aus dem Absorbens entfernten diacetylenhaltigen Gases eignet sich vorteilhaft Erdgas, aber auch andere Gase wie Kohlenwasserstoffe, CO, Synthesegas, Armgas, N₂ oder Gemische dieser Gase sind geeignet.

Das in bezug auf die Selbstzersetzlichkeit des Diacetylens inertisierte Gasgemisch enthält bevorzugt 55-85 Vol-% Inertgas, 1 bis 30 Vol.-% Diacetylen und 10-20 Vol.-% andere Bestandteile wie Acetylen, Vinylacetylen und Benzol. Der Anteil des Diacetylens im inertisierten Gasgemisch beträgt insbesondere 5 bis 20 %. Die Obergrenze wird durch die Grenze des explosiven Selbstzerfalls im Inertgas bestimmt. Ein typisches Gasgemisch hat folgende Zusammensetzung: 58 % CH₄, 18 % Diacetylen, 5 % Stickstoff, 4 % Acetylen, 4,5 % Vinylacetylen, 4 % Benzol, 2 % C₂H₆, 2 % Cyclopentadien, 2,5 % Restbestandteile.

Die reversible Absorption des Diacetylens wird in besonders bevorzugter Weise so durchgeführt, daß das Diacetylen durch eine Vorwäsche des das Gesamtacetylen enthaltenden Spaltgases mit der polaren Flüssigkeit als Absorbens, Entgasen des Absorbens in einem Vakuumstripper, anschließendes Auswaschen des noch im Gasstrom befindlichen Absorbens mit Wasser und Abkühlen des diacetylenhaltigen Gases abgetrennt wird.

Die Umsetzungstemperatur für die Reaktion des Diacetylens mit den Hydrazinen liegt vorteilhaft bei -40°C bis 150°C, wobei Temperaturen von 50°C bis 120°C besonders geeignet sind, eine gute Ausbeute bei nahezu vollständigem Diacetylenumsatz zu erzielen.

Die Umsetzung kann bei leichtem Unter- wie Überdruck ausgeführt werden. Höhere Kompression ist aus Sicherheitsgründen nicht ratsam. Die besten Ergebnisse erzielt man bei Normaldruck.

Die Umsetzung des Teilstroms mit Hydrazinen oder deren Lösungen kann sowohl diskontinuierlich (Batchverfahren) als auch kontinuierlich (Gleich- oder Gegenstrom) gestaltet werden. Bei Nutzung eines gasförmigen Teilstroms sind Verfahren, die bekanntermaßen eine gute Gasverteilung in Flüssigkeiten erzielen, z.B. die Verwendung von Apparateteilen wie Begasungsring, Lochboden, Sprühreaktor/Absorberturm, vorteilhaft anzuwenden.

Das Gasgemisch aus der Spaltgasaufbereitung hat gewöhnlich gegenüber der Umgebung leicht erhöhte Betriebstemperatur. Die Vorabscheidung von leicht kondensierbaren Gasbestandteilen in Abscheidebehältern in der Reaktorzuleitung bei Umgebungstemperatur verbessert die Reinheit des Rohproduktes.

Die Hydrazine werden vorteilhaft in reiner Form oder in Form wäßriger Lösungen eingesetzt. Aber auch Lösungen von Hydrazinen in anderen protischen oder polaren aprotischen Lösungsmitteln, z.B. Alkohole, Säureamide, Lactame, alkylierte Harnstoffe, wie sie bereits weiter oben als polare Flüssigkeiten beschrieben sind, sowie Ester, Lactone und Glykolether, insbesondere Ethylenglykoldiethylether, oder weniger polaren Lösungsmitteln wie Ethern, Aromaten und Kohlenwasserstoffen sind möglich.

Der Reaktionsschritt b) des erfindungsgemäßen Verfahrens wird bevorzugt und technisch unproblematisch unter Rückführung der nicht umgesetzten Anteile an Hydrazinen in die Reaktion durchgeführt.

In den folgenden Beispielen ist das inertisierte, diacetylenhaltige Gasgemisch mit HA-Gas und Diacetylen mit DA bezeichnet. Das diacetylenhaltige Absorbat aus der Spaltgasvorwäsche wird mit Vorwaschlösung (+ Angabe des Lösungsmittels) benannt. Die diacetylenhaltige Lösung aus dem Sumpf der HA-Gaskühler wird mit BTX-Lösung abgekürzt. Das Diacetylen im HA-Gas bzw. im Abgas nach der

Reaktion wurde gaschromatisch auf einer gepackten Säule (20 % Reoplex 400 auf Chromosorb PAW) mit Trägergas N₂ (35 ml/min) und FID-Detektion bestimmt. Die Konzentrationen sind in Vol.-% angegeben.

Die Diacetylenbestimmung in flüssiger Phase sowie die Ermittlung der Methylpyrazol- und Hydrazinkonzentrationen erfolgte gaschromatographisch auf einer Kapillarsäule HP1 mit einem WLD-Detektor.

### Beispiel 1

In eine mit 1000 g Hydrazinhydrat (64 Gew.-% Hydrazin) gefüllte und auf 100°C beheizte 2-1-Blasensäule wurden durch eine Glasfritte (D2) am Boden 100 l/h HA-Gas eingeleitet.

Dieses HA-Gas wurde in einem mehrstufigen Reinigungsprozeß aus dem Spaltgas der Acetylenproduktion erhalten, indem die höheren Acetylene durch eine Wäsche des Spaltgases mit wenig NMP bei 35°C und ca. 10 bar herausgelöst werden, die Waschlösung anschließend bei ca. 110°C mit Wasserdampf bei Unterdruck (0,13 bar) gestrippt und das resultierende Strippgas in der Folge mit Kühlwasser gewaschen (Rückgewinnung von NMP) und auf ca. 20 bis 40°C gekühlt (Schwersiederabscheidung) wurde. Schließlich stellt man (vor und nach der Kompression auf ca. 1,3 bar) durch Zudosieren von Naturgas auf einen Diacetylengehalt von 14 bis 18 Vol.-% ein. In einem wassergekühlten Abscheider wurde das HA-Gas letztlich auf eine Betriebstemperatur von ca. 40°C temperiert.

Bei einer durchschnittlichen Abreicherung des Diacetylens von über 90 % wurden innerhalb von 18 h 88 % des Hydrazins umgesetzt. Bei der destillativen Aufarbeitung (bei Normaldruck wie Vakuum möglich) wurden 1290 g 3-Methylpyrazol (3-MP) (90 % Ausbeute, bezogen auf umgesetztes Hydrazin) mit einer Reinheit > 99,4 % isoliert.

### Beispiel 2

In einer Rieselkolonne wurden 110 g 25%iges Hydrazinhydrat bei 60°C im Kreis gefahren. Im Gegenstrom wurden 10 l/h HA-Gas (HA-Gasgewinnung wie in Beispiel 1) eingeleitet, dessen DA-Gehalt zwischen 4 und 13 % schwankte. Nach 20 h Reaktionszeit hatte sich bei einem Hydrazinumsatz von 20 % in 95%iger Selektivität (GC-Analyse des Rohaustrags) 3-MP gebildet.

### Beispiel 3

Wie in Beispiel 1 wurden 100 g Hydrazinhydrat (64%ig) mit HA-Gas bei 80°C und einer Gasdosiergeschwindigkeit von 50 l/h umgesetzt. Der DA-Gehalt schwankte zwischen 5 und 12 %. Nach 40 h war ein Hydrazinumsatz von 85 % erreicht. Durch Destillation wurden 126 g 3-MP (90 % Ausbeute, bezogen auf umgesetztes Hydrazin) erhalten.

### Beispiel 4

In einer 5-l-Blasensäule wurden 2500 g Hydrazinhydrat (64%ig) bei 60°C mit HA-Gas umgesetzt. Das HA-Gas mit einem Diacetylengehalt von 8 bis 13 % wurde mit einer Gasdosiergeschwindigkeit von 150 bis 180 l/h zudosiert. Die durchschnittliche DA-Abreicherung betrug 92 % über 35 h. Die fraktionierte Destillation erlaubte die Rückgewinnung von 650 g unumgesetztem Hydrazin in wäßriger Lösung. 2050 g 3-MP (84 % Ausbeute, bezogen auf umgesetztes Hydrazin) wurden in 99,5%iger Reinheit erhalten.

### Beispiel 5

In der Apparatur von Beispiel 4 wurden 2000 g Hydrazinhydrat bei 80°C mit HA-Gas versetzt bis zur völligen Umsetzung des Hydrazins. Die DA-Konzentration im HA-Gas schwankte zwischen 6 und 15 %. Bei einer Gaseinleitungsgeschwindigkeit von 150 l/h lag die DA-Abreicherung bis zu einem Umsatz des Hydrazins von 88 % über 85 %. Nach 80 h waren 99,4 % des eingesetzten Hydrazins umgesetzt. Die fraktionierte Aufdestillation ergab 7 g Hydrazin (in wäßriger Lösung) und 3009 g 3-MP (91,8 % Ausbeute, bezogen auf eingesetztes Hydrazin).

### Beispiel 6

In der Apparatur von Beispiel 1 wurde in 1000 g Hydrazinhydrat (64%ig) 80 l/h HA-Gas bei 60°C eingeleitet. Die DA-Konzentration am Einlaß schwankte von 12 bis 14 %. Bei einer durchschnittlichen DA-Abreicherung des HA-Gases von 70 % wurden in 30 h 80 % des eingesetzten Hydrazins umgesetzt. Die fraktionierte Destillation lieferte 127 g Hydrazin (in wäßriger Lösung) und 1248 g 3-MP (95 % Ausbeute, bezogen auf umgesetztes Hydrazin).

### Beispiel 7

In der Apparatur aus Beispiel 1 wurden 1000 g Hydrazinhydrat (64%ig) bei Raumtemperatur (20°C) mit 100 l/h HA-Gas durchströmt. Es wurde eine Selbsterwärmung der Reaktionslösung auf 37°C beobachtet. Die Einlaßkonzentration des DA schwankte zwischen 12 und 19 %. In 35 h setzten sich 70 % des Hydrazins um bei einer durchschnittlichen DA-Abreicherung von 75 %. Die destillative Auftrennung des Rohaustrages ergab 1025 g 3-MP (89,3 % Ausbeute, bezogen auf umgesetztes Hydrazin) in 99,6%iger Reinheit.

### Beispiel 8

2000 g Hydrazinhydrat (64 %ig) wurden bei 80°C mit 120 l/h HA-Gas durchströmt. Der DA-Gehalt am Einlaß schwankte zwischen 11 und 17 %. Nach 30 h Betriebszeit waren 82 % des eingesetzten Hydrazins verbraucht. Die fraktionierte Destillation lieferte 234 g (18 %) nichtumgesetztes Hydrazin als wäßrige Lösung mit einem Gehalt von 35 % 3-Methylpyrazol (198 g). Als Reinprodukt (Reinheit > 99 %) destillierten 2295 g 3-Methylpyrazol über. Die Gesamtausbeute an 3-Methylpyrazol aus beiden Teilströmen betrug somit 92,9 % (bezogen auf umgesetztes Hydrazin).

Der hydrazinhaltige Teilstrom (565 g) wurde mit 64 %igem Hydrazinhydrat auf eine Menge von 1 000 g ergänzt (Gesamthydrazingehalt 41,4 %). Bei 80°C wurden binnen 15-stündiger Einleitung von 120 l/h HA-Gas (DA-Gehalt von 13 bis 16,5 %) 83 % des eingesetzten Hydrazins umgesetzt. Durch fraktionierte Destillation wurden 1251 g 3-Methylpyrazol isoliert (97 % Ausbeute, bezogen auf umgesetztes Hydrazin).

### Beispiel 9

471 g BTX-Lösung mit einem DA-Gehalt von 5,6 % wurden in einem Rührgefäß mit Rückflußkühler mit 50 g N₂H₄ x H₂O versetzt und unter Rühren zum gelinden Sieden erhitzt. Nach einer Stunde war kein Diacetylen mehr detektierbar. Der 3-Methylpyrazolgehalt betrug 7,5 % entsprechend einer Ausbeute von ca. 85 %. Bei der destillativen Auftrennung wurde als erste Fraktion bei einer Temperatur von 80 bis 100°C unter Normaldruck ein Gemisch der BTX-Aromaten und Wasser erhalten. Nach Phasentrennung des Kondensats resultierten 420 g diacetylenfreies BTX.

### Beispiel 10

In der Apparatur aus Beispiel 1 wurde ein Gemisch aus 500 g Hydrazinhydrat und 500 g Dimethylformamid (DMF) bei 60°C mit 100 l/h HA-Gas durchströmt. Die Einlaßkonzentration des DA schwankte zwischen 12 und 19 %. Nach 12 Stunden hatten sich 80 % des eingesetzten Hydrazins umgesetzt. Die durchschnittliche Abreicherung des DA aus dem HA-Gas betrug über 85 %. Die fraktionierte Destillation lieferte 610 g reines 3-MP (93 % Ausbeute, bezogen auf umgesetztes Hydrazin).

### Beispiel 11

In einem 100 ml Rührgefäß mit Rückflußkühlung wurden 50 g einer einer Spaltgasvorwaschlösung entsprechenden 2,2%igen Diacetylenlösung in MeOH bei 60°C mit 5 g (0,1 mol) Hydrazinhydrat versetzt. Nach 2 h war kein Diacetylen mehr detektierbar. 3-Methylpyrazol hatte sich in einer Selektivität von 82 %, bezogen auf das eingesetzte Hydrazin, gebildet.

### Beispiel 12

In einer Apparatur nach Beispiel 11 wurden 5 g Hydrazinhydrat bei 80°C mit 50 g der methanolischen Vorwaschlösung versetzt. Nach beendeter Zugabe wurde noch 2 h bei 70°C am Rückfluß gekocht. Bei vollständigem Diacetylenumsatz bildete sich 3-Methylpyrazol in, bezogen auf Hydrazin, 81%iger Selektivität.

### Beispiel 13

In eine Absorberapparatur mit einem Durchmesser von 40 mm, einer Höhe von 320 mm und einer geschütteten Füllung aus 3 mm Glasraschigringen wurden 250 l/h HA-Gas mit einem von 6,6 bis 14,8 % schwankenden Diacetylengehalt unten durch einen Glaslochboden eingeleitet. Dem Gasstrom wurde ein Flüssigkeitsstrom von zu Beginn 64 %igem N₂H₄·H₂O mit einer Umlaufgeschwindigkeit von 52 l/h entgegengefahren. Die Lösung wurde komplett im Kreis geführt und das Hydrazin abgereichert. Über den Hydrazinkonzentrationsbereich von 64 bis ca. 15 % in der Flüssigphase wurde eine Raumzeitausbeute an 3-MP von 123 g/lh erzielt.

### Beispiel 14

In einer Blasensäule (Durchmesser 2 cm) mit einer Gaseinleitungsfritte aus Glas (40 - 90 µm Porendurchmesser, D 2) wurden eine Mischung aus 90 g Methylhydrazin und 60 g H₂O vorgelegt und auf 80°C erhitzt. 20 l/h eines Teilstroms des HA-Abgases der Acetylenproduktion wurden während 18 Betriebsstunden durch die Reaktionslösung hindurchgeleitet. Die Diacetylenkonzentration des HA-Gases am Reaktoreingang schwankte von 11 - 15 %. Bei einer durchschnittlichen Abreicherung des Diacetylens von über 80 % wurden 127 g (66 % Ausbeute) 1,5-Dimethylpyrazol und 35 g 1,3-Dimethylpyrazol (18 % Ausbeute) erhalten.

### Beispiel 15

In einer 500 ml-Rührapparatur mit Rückflußkühler wurde eine Lösung von 18,8 g Phenylhydrazin in 50 g N-Methylpyrrolidon vorgelegt und auf 60°C erwärmt. Über 24 h wurden zu dieser Lösung insgesamt 100 ml HA-haltige Vorwaschlösung (Diacetylengehalt ca. 8,5 % in NMP) aus der Spaltgasaufbereitung einer Acetylenanlage zudosiert. Die Lösung verfärbte sich zusehends dunkler und enthielt nach Reaktionsende 12 % der beiden Isomeren 1-Phenyl-3(5)-methylpyrazole (ca. 80 % Ausbeute).

## Patentansprüche

1. Verfahren zur Herstellung von Methylpyrazolen der allgemeinen Formeln Ia und Ib 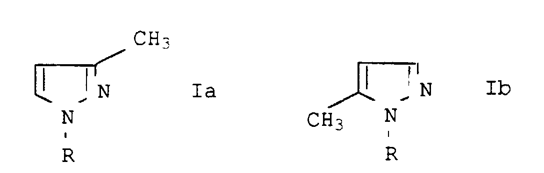 in der R für Wasserstoff, einen C₁-C₆-Alkylrest oder einen gegebenenfalls substituierten Phenylrest steht, aus Diacetylen und Hydrazinen, dadurch gekennzeichnet, daß
a) aus dem bei der Acetylenerzeugung resultierenden Spaltgas ein Teilstrom, der das Diacetylen enthält, durch reversible Absorption in einer polaren Flüssigkeit oder einem Gemisch höhersiedender Kohlenwasserstoffe abgetrennt wird und gegebenenfalls mit einem Verdünnungsmittel verdünnt wird, und
b) dieser Teilstrom ohne weitere physikalische oder chemische Behandlung mit Hydrazinen der allgemeinen Formel II
RHN-NH₂ II
in der R die oben angegebene Bedeutung hat, bei Temperaturen von (-40) bis +150°C und Normaldruck umgesetzt wird.

2. Verfahren nach Anspruch 1, dadurch gekennzeichnet, daß die polare Flüssigkeit oder der Kohlenwasserstoff Wasser, ein Alkohol, ein Keton, Benzol, Toluol, Xylol, Dimethylformamid, N-Methylpyrrolidon oder ein Gemisch davon ist.

3. Verfahren nach Anspruch 1, dadurch gekennzeichnet, daß das Inertgas ein Gemisch leichtsiedender Kohlenwasserstoffe, Wasserstoff, Kohlenmonoxid, Stickstoff, Erdgas, Acetylen oder ein Gemisch dieser Gase ist.

4. Verfahren nach Anspruch 1, dadurch gekennzeichnet, daß die polare Flüssigkeit N-Methylpyrrolidon ist.

5. Verfahren nach Anspruch 1, dadurch gekennzeichnet, daß das Inertgas Erdgas ist.

6. Verfahren nach Anspruch 1, dadurch gekennzeichnet, daß der Anteil an Diacetylen nach der Verdünnung mit Inertgas 5 bis 30 Vol.-% beträgt.

7. Verfahren nach Anspruch 1, dadurch gekennzeichnet, daß die Hydrazine in reiner Form, in wäßriger Lösung oder als Lösungen in protischen oder aprotischen Lösungsmitteln eingesetzt werden.

8. Verfahren nach Anspruch 1, dadurch gekennzeichnet, daß nicht umgesetztes Hydrazin in die Reaktion zurückgeführt wird.

9. Verfahren nach Anspruch 1, dadurch gekennzeichnet, daß die reversible Absorption des Diacetylens gemäß Verfahrensschritt a) durch eine Vorwäsche des das Gesamtacetylen enthaltenden Spaltgases mit der polaren Flüssigkeit, Entgasen des Absorbens in einem Vakuumstripper, anschließendes Auswaschen des noch im Gasstrom befindlichen Absorbens mit Wasser und Abkühlen des diacetylenhaltigen Gases erfolgt.

## Claims

1. A process for preparing methylpyrazoles of the general formulae Ia and Ib 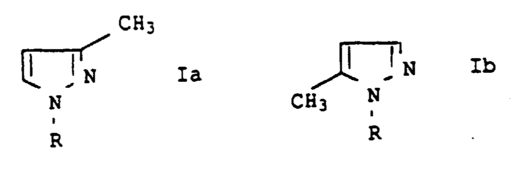 where R is hydrogen, C₁-C₆-alkyl or unsubstituted or substituted phenyl, from diacetylene and hydrazines, wherein
a) a part stream which contains the diacetylene is removed from the product gas resulting from acetylene production by reversible absorption in a polar liquid or a mixture of high-boiling hydrocarbons, and is, where appropriate, diluted with a diluent, and
b) this part stream is reacted without further physical or chemical treatment with hydrazines of the general formula II
RHN-NH₂ II
where R has the abovementioned meaning, at from -40 to +150°C under atmospheric pressure.

2. A process as claimed in claim 1, wherein the polar liquid or the hydrocarbon is water, an alcohol, a ketone, benzene, toluene, xylene, dimethylformamide, N-methylpyrrolidone or a mixture thereof.

3. A process as claimed in claim 1, wherein the inert gas is a mixture of low-boiling hydrocarbons, hydrogen, carbon monoxide, nitrogen, natural gas, acetylene or a mixture of these gases.

4. A process as claimed in claim 1, wherein the polar liquid is N-methylpyrrolidone.

5. A process as claimed in claim 1, wherein the inert gas is natural gas.

6. A process as claimed in claim 1, wherein the diacetylene content after the dilution with inert gas is from 5 to 30% by volume.

7. A process as claimed in claim 1, wherein the hydrazines are employed in pure form, in aqueous solution or as solutions in protic or aprotic solvents.

8. A process as claimed in claim 1, wherein unreacted hydrazine is returned to the reaction.

9. A process as claimed in claim 1, wherein the reversible absorption of the diacetylene in process step a) takes place by a prewash of the product gas containing all the acetylene with the polar liquid, degassing the absorbent in a vacuum stripper, subsequently washing out the absorbent which is still present in the gas stream with water, and cooling the diacetylene-containing gas.

## Revendications

1. Procédé de préparation de méthylpyrazoles des formules générales Ia et Ib 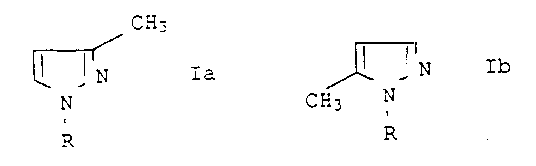 dans lesquelles R représente un atome d'hydrogène, un radical alkyle en C₁ à C₆, ou un radical phényle éventuellement substitué, à partir du diacétylène et d'hydrazines, caractérisé en ce que
a) à partir du gaz de scission résultant de la fabrication de l'acétylène, on sépare un courant partiel, qui contient le diacétylène, par absorption réversible dans un liquide polaire ou un mélange d'hydrocarbures à points d'ébullition élevés et on le dilue éventuellement avec un diluant et
b) on fait réagir ce courant partiel sans autre traitement physique ou chimique supplémentaire avec des hydrazines de la formule générale II
RHN-NH₂ II
dans laquelle R possède les significations qui lui ont été attribuées ci-dessus, à des températures de -40°C à +150°C et à la pression normale.

2. Procédé suivant la revendication 1, caractérisé en ce que le liquide polaire ou l'hydrocarbure est l'eau, un alcool, une cétone, le benzène, le toluène, le xylène, le diméthylformamide, la N-méthylpyrrolidone, ou un mélange de ceux-ci.

3. Procédé suivant la revendication 1, caractérisé en ce que le gaz inerte est un mélange d'hydrocarbures à bas points d'ébullition, de l'eau, du monoxyde de carbone, de l'azote, du gaz naturel, de l'acétylène ou un mélange de ces gaz.

4. Procédé suivant la revendication 1, caractérisé en ce que le liquide polaire est la N-méthylpyrrolidone.

5. Procédé suivant la revendication 1, caractérisé en ce que le gaz inerte est le gaz naturel.

6. Procédé suivant la revendication 1, caractérisé en ce que la fraction en diacétylène après la dilution avec le gaz inerte varie de 5 à 30% en volume.

7. Procédé suivant la revendication 1, caractérisé en ce que les hydrazines se mettent en oeuvre sous forme pure, en solution aqueuse ou sous la forme de solutions dans des solvants protique ou aprotiques.

8. Procédé suivant la revendication 1, caractérisé en ce que l'on renvoie l'hydrazine non convertie à la réaction.

9. Procédé suivant la revendication 1, caractérisé en ce que l'absorption réversible du diacétylène selon l'étape opératoire a) s'effectue par un prélavage du gaz de scission contenant l'acétylène total avec le liquide polaire, dégazage de l'absorbant dans un laveur sous vide, lixiviation subséquente de l'absorbant qui se trouve encore dans le courant de gaz avec de l'eau et refroidissement du gaz contenant le diacétylène.
